Europäisches Patentamt

European Patent Office

Office européen des brevets

(19)

(11) Publication number: 0 211 592
A2

(12) **EUROPEAN PATENT APPLICATION**

(21) Application number: 86305794.9

(22) Date of filing: 28.07.86

(51) Int. Cl.⁴: **A61K 37/54** , A61K 9/08 , A61K 9/14 , A61K 47/00

(30) Priority: 29.07.85 US 759785

(43) Date of publication of application:
25.02.87 Bulletin 87/09

(84) Designated Contracting States:
AT BE CH DE FR GB IT LI LU NL SE

(71) Applicant: SMITHKLINE BECKMAN
CORPORATION
One Franklin plaza
Philadelphia Pennsylvania 19103(US)

(72) Inventor: **Malefyt, Thomas Robert**
402 Hanover Court
Wayne Pennsylvania 19087(US)
Inventor: **Meyers, Chester Allen**
425 Pine Drive
Phoenixville Pennsylvania 19460(US)

(74) Representative: **Waters, David Martin, Dr. et al**
Smith Kline & French Laboratories Ltd.
Patent Department Mundells
Welwyn Garden City Hertfordshire AL7
1EY(GB)

(54) **Pharmaceutical dosage unit.**

(57) A pharmaceutical dosage unit of human tissue Plasminogen Activator (tPA) comprising tPA in a buffered solution at pH 3.5 -5.5, and its use in thrombolytic therapy.

EP 0 211 592 A2

## Pharmaceutical Dosage Unit

This invention relates to a pharmaceutical formulation for parenteral administration and more particularly to a pharmaceutical dosage unit for thrombolytic therapy.

Human tissue Plasminogen Activator (tPA) is a glycoprotein of about 70,000 daltons and 527 amino acids. It is secreted by a variety of human tissues and is naturally present in serum. tPA functions in clot lysis by converting plasminogen to plasmin. tPA has an affinity for and is more active in a presence of fibrin. Thus, tPA is useful in treating disorders associated with thrombi formation such as deep vein thrombosis, acute myocardial infarction and pulmonary embolism. See, generally, Rijken et al., Biochem. Biophys. Acta580:140 (1979), Vetterlein et al.,J. Biol. Chem.254:575 (1979), Rijken et al., J. Biol. Chem.256:7035 (1981) and Matsuo et al., Nature 291:590 (1981).

tPA is overproduced by certain cell lines, including the Bowes melanoma cell line. See, e.g., Wilson et al.,Canc. Res.40:933 (1980). Collen et al., EP-A-41,776, disclose purification of Bowes melanoma-derived tPA. Wilson, EP-A-113,319, disclose production and purification of tPA using a serum-independent Bowes melanoma cell line.

tPA can also be prepared by recombinant DNA techniques. Isolation of mRNA for tPA is disclosed, e.g., by Opdenakker et al., Eur .J. Biochem. 121:269 (1982). Isolation of cDNA for part of tPA is disclosed by Edlund et al., Proc. Natl. Acad. Sci. USA80 :349 (1983). Cloning of cDNA for tPA in E.coli is disclosed by Pennica et al., Nature301:214 (1983). Cloning of cDNA for tPA in E. coli and in Chinese Hamster Ovary cells is disclosed by Goeddel et al., EP-A-93,619 and by. Levinson et al., EP-A-117,059.

Brouty-Boye, Bio/Technology, December 1984, p, 1058, report production of tPA by human embryonic lung cells.

Robinson, WO84-01786, discloses a modified tPA lacking all or a portion of the carbohydrate moieties present in native tPA.

tPA is not highly soluble in aqueous solution. Its solubility in water at pH 7.0 is about 0.1 mg/ml. Thus, tPA is commonly solubilized with typical solubilizing agents during purification. These include chaotropic denaturing agents such as urea and thiocyanate, surfactants such as detergents, carbohydrates such as mannitol, and proteins such as albumin and gelatin. Although these agents apparently improve the water solubility of tPA, resulting tPA solutions tend to be less stable, are not recognized as being suitable for parenteral administration or must be employed in unacceptably large amounts.

Wilson, EP-A-113-319, suggest addition of a non-ionic detergent to prevent adsorption to container surfaces and to improve stability. An illustrative pharmaceutical composition disclosed by Wilson contains .075 mg/ml of tPA in 0.3 M sodium chloride and 0.01% Tween® 80.

Goeddel et al., Ep-A-93,619, disclose that tPA can be formulated by standard techniques and suggest, as an example, a vial containing 25,000 International Units of tPA, 25 mg of mannitol and 45 mg of NaCl reconstituted with 5 ml of water and mixed with 0.9% sodium chloride.

Collen et al., EP-A-41,766, state that tPA can be formulated by standard techniques with or without additives such as sodium chloride, glucose, mannitol and albumin. A constructively exemplified pharmaceutical composition contains tPA, 0.001% Tween® 80 and 0.01-1% of albumin or mannitol in phosphate buffered saline.

Yoshizaki et al., EP-A-112,940, disclose use of albumin as a stabilizer for tPA.

Murakami et al., EP-A-123,304, disclose stabilization of tPA by addition of gelatin.

Kruithof et al., Biochem. J.226:631-636 (1985), disclose a purification scheme for tPA produced by serum-independent Bowes melanoma cells which included the steps of centrifuging conditioned medium, adding Tween® 80 and NaN₃ to the supernatant, adjusting the pH to 4.5 with 6 M HCl, passing the solution over a SP-Sephadex® column and eluting the tPA with NaCl in a sodium acetate buffer, at pH 4.5 (0.1 M sodium acetate, 0.25 M NaCl, 0.01% Tween® 80, 0.05% NaN₃). Eluate fractions were pooled, concentrated to 0.93 mg/ml of tPA (42,800 units/ml), passed over a column of Sephadex® G-100 in a sodium acetate buffer (50 mM sodium acetate, 0.5 M NaCl, 0.01% Tween® 80, 0.05% NaN₃, pH 4.5). The product was concentrated to 0.38 mg/ml of tPA (35,000 units/ml) and stored at -70°C.

American Diagnostica, Inc. sells lyophilized tPA. The vendor's instructions for reconstitution are, "Use 0.1 M acetic acid/sodium acetate buffer, pH 3.9, then bring up the pH, as desired. Albumin, 1 mg/ml, can be added to avoid precipitation."

It is an object of the present invention to prepare a pharmaceutical formulation for use in preparing a sterile, pharmaceutical dosage unit of tPA which does not require typical solubilizing/stabilizing agents such as chaotropic agents, surfactants, proteinaceous agents and carbohydrates to achieve high solubility, that is, greater than the solubility in water, 0.1 mg/ml.

It is a further object of the present invention to prepare a pharmaceutical formulation of tPA in which the tPA is relatively stable at ambient temperature.

It is yet a further object of the present invention to prepare a pharmaceutical formulation of tPA which is suitable for parenteral administration.

Obtainment of these and other objects of the invention are fully disclosed herein below.

In one aspect, the invention is a sterile pharmaceutical dosage unit of tPA for parenteral administration which comprises tPA in a buffered, pharmaceutically acceptable solvent at pH 3.5-5.5.

In another aspect, the invention is a kit which comprises one or more sterile containers of tPA in lyophilized form and one or more other sterile containers of solution for reconstitution, wherein a buffer is provided in one or both containers and the buffer, the solution for reconstitution and the amounts of the tPA and of the solution for reconstitution are selected so as to provide upon combining the contents of said containers, a reconstituted product which is a pharmaceutical dosage unit for parenteral administration comprising 0.1 to 15 mg/ml of tPA in a buffered, pharmaceutically acceptable solvent at pH 3.5-5.5.

The Figure is a graphic illustration of initial degradation (loss of activity) rates of tPA, 0.39 mg/ml except where otherwise indicated, in urea or acetic acid/ammonium acetate solutions.

It has now been discovered that tPA is highly soluble when buffered at pH 3.5-5.5, in an absence of solubilizing/stabilizing agents. For example, at pH 4.0 (0.1 M ammonium acetate), the solubility of tPA is greater than about 15 mg/ml; at pH 5.0 (0.1 M ammonium acetate), the solubility of tPA is approximately 3 mg/ml. As stated previously, the solubility of tPA in water at pH 7.0 is about 0.1 mg/ml.

The solubility of tPA in the formulation of this invention at pH 4.0 is comparable to the solubility which is obtainable through use of 2 M urea at pH 7.5. However, in the formulation of the invention, presence of such solubilizing agent, which is an osmotic diuretic and may cause undesirable side effects when co-administered with tPA, is not required to obtain the high solubility.

Another manifestation of the display disclosed and claimed herein is that in the pH range indicated, tPA is highly stable. Such enhanced stability is apparent at low as well as high concentrations of tPA in the pharmaceutical dosage unit of the invention. For example, the table which follows reports the percent activity of tPA at low concentration (0.39 mg/ml, except where otherwise indicated), at 40°C in urea (2 M urea, 0.3 M NaCl, 20 mM tris-HCl and 0.01% Tween® 80, pH 7.5) or 100 mM ammonium acetate at pH 3.5-5.5 in the S-2251 assay. The S-2251 assay is described by Weinberg et al., J. Immunol. Meth.75:289 (1984). A high temperature was employed to expedite loss of activity, in accordance with standard practice.

| | Urea | | Ammonium Acetate | | | | | |
|---|---|---|---|---|---|---|---|---|
| Days | pH 7.5* | pH 7.5 | pH 3.5 | pH 4.0 | pH 4.5 | pH 5.0 | pH 5.5 | pH 4.0** |
| 0 | 100 | 100 | 100 | 100 | 100 | 100 | 100 | 100 |
| 1 | 67 | 88 | | | | | | 65 |
| 2 | | 51 | 84 | 84 | 80 | 94 | 92 | |
| 3 | 19 | 24 | 71 | 90 | 77 | 90 | 84 | |
| 4 | | 13 | | | | | | 66 |
| 5 | 24 | | | | | | | |
| 6 | | 20 | | | | | | |
| 7 | | 2 | 53 | 62 | 81 | 82 | 65 | |
| 8 | 9 | 4 | | | | | | |
| 9 | | | 63 | 62 | 81 | 62 | 72 | |
| 10 | 4 | | | | | | | |
| 11 | 37 | | 37 | 56 | 82 | 69 | 69 | 52 |
| 12 | | | | | | | | |
| 13 | 18 | | | | | | | |
| 14 | | | 34 | 40 | 70 | 62 | 60 | |
| 15 | 17 | | | | | | 23 | |
| 16 | | | 22 | 16 | 53 | 58 | 24 | |
| 18 | | | 15 | 9 | 63 | 55 | | |

*1.3 mg/ml   **9.8 mg/ml

The above results are graphically illustrated in the Figure. The results are consistent with electrophoretic analyses of tPA stored in the 100 mM acetate solution. Over a period of 3 weeks at 40°C, no degradation of the tPA in the 100 mM ammonium acetate solution was detected at pH 4.0, 4.5, 5.0 and 5.5 by polyacrylamide gel electrophoresis; small bands corresponding to 12, 16 and 21 kilodaltons appeared after 3 weeks at 40°C at pH 3.5.

Another aspect of this invention is that although tPA is largely inactive below about pH 7, it has been found that activity is restored upon restoration of about pH 7. Thus, upon administration to a patient in need of thrombolytic therapy, the environment of the tPA in the dosage unit of the invention is changed to pH 7 and the tPA becomes active.

The stability of the pharmaceutical dosage unit of the invention is increased at low temperature. Thus, it is preferably stored at no greater than room temperature, about 20°-25°C. Although tPA in the formulation of the invention is more stable at low temperature, e.g., 0 to 15°C, an advantage of the pharmaceutical dosage unit of the invention is its stability at higher temperature, e.g., >20°C, as evidenced by the results reported above. At 4°C, tPA in a 2M urea solution (pH 7.5) and tPA in a 100 mM ammonium acetate solution (pH 4.0) both retained substantially all activity after several weeks. At room temperature (about 22°C), tPA in the formulation of the invention will be stable for several weeks. If storage for longer periods is desired, tPA in an appropriate vehicle can be lyophilized for storage, and then reconstituted to form a pharmaceutical dosage unit of the invention for immediate use or for shorter term storage.

The results referred to hereinabove were obtained using tPA produced by recombinant Chinese hamster ovary (CHO) cells grown in a presence of serum. The invention, however, includes pharmaceutical dosage units comprising tPA from other sources, including serum, melanoma cells, serum-independent mammalian cells, bacteria, yeast, insect cells, plant cells and other mammalian cells, recombinant or native. The invention also pertains to other forms of tPA including, for example, single chain tPA, variant tPA, i.e., tPA in which one or a few amino acids have been altered without significant affect on the activity, solubility and stability of such variant tPA, and tPA in which glycosyl residues have been modified.

In addition to ammonium acetate, other pharmaceutically acceptable buffering agents can be employed in the pharmaceutical dosage unit of the invention. Included among useful buffers are mixtures of the following acids and their salts: acetic acid, adipic acid, citric acid, lactic acid, tartaric acid, aspartic acid and glutamic acid. Other acids which buffer in the desired range and which may be useful in the invention include, among others, glutaric acid, maleic acid, fumaric acid, succinic acid, pimelic acid, beta-alanine and enol pyruvic acid. The preferred anion is acetate. Preferred cations are sodium and ammonium.

The concentration of buffer in the pharmaceutical dosage unit of the invention is selected to maintain the pH at 3.5-5.5, preferably 4.0-5.0 and more preferably 4.4-4.8. Typically, the concentration of buffer is about 10 to 200 mM, preferably 20 to 100 mM, of a sodium or ammonium salt of a suitable acid. The buffers are prepared by standard techniques, e.g., adding NaOH to 100 mM acetic acid in water to a selected pH within the range useful in the invention for use in preparing a pharmaceutical dosage unit of the invention in which the buffer is 100 mM sodium acetate.

Preferably, the pharmaceutical formulation of the invention is an aqueous solution. However, certain polar, water-miscible, non-aqueous solvents can be used for parenteral formulations. Such solvents may be used in the instant invention provided the activity and stability of the tPA is not significantly adversely impacted. Which of such solvents are useful can be determined by routine experimentation.

In addition to tPA and buffer, the pharmaceutical dosage unit of the invention can contain small amounts of other agents suitable for parenteral administration, e.g., a preservative such as thimerosal, chlorobutanol or methyl paraben, and an agent to adjust tonicity such as sodium chloride, dextrose or mannitol. The pharmaceutical dosage unit of the invention is preferably isotonic (250 to 310 mOSM/kg). Wang et al., J. Parenteral Drug Assoc. 34:452(1980), report excipients which have been used in parenteral formulations. In addition, the pharmaceutical dosage unit of the invention can include additional pharmaceutically active agents such as fibrinolytic, cardiotonic, antiarrythmic or hypotensive agents.

The pharmaceutical stability of the tPA in the pharmaceutical dosage unit of the invention can be further enhanced by inclusion therein of a hydrophilic polymeric cryoprotective agent, such as hydroxy alkyl cellulose, gelatin, acacia gum, polyvinylpyrrolidone (e.g., molecular weight 10,000 to 60,000) and polyalkylene glycols, such as polyethylene glycol (e.g., molecular weight 4,000 to 40,000). Use of such agent increases stability (that is, minimizes loss of activity and protein degradation) in solution, on lyophilization and upon reconstitution following lyophilization. A preferred such agent is polyvinylpyrrolidone (PVP) having a molecular weight of 20,000 to 50,000

Such agent can be added to a tPA formulation in solution or in powdered form. Preferably, such agent is added to a tPA formulation of the invention prior to lyophilization so that upon reconstitution, the amount of the agent in the final, reconstituted dosage unit is a safe and effective amount. Such amount in the case of polyvinylpyrrolidone, molecular weight 40,000 (Plasdone C-30), is about 1 to 15 mg of PVP, preferably 4 to 10 mg, per 1 mg of tPA. In the case of gelatin, larger amounts may be preferred to achieve increased stability, e.g., 10 to 1,000 mg of gelatin per 1 mg of tPA.

The pharmaceutical dosage unit of the invention contains 0.1 to 15 mg/ml of tPA, preferably 0.5 to 10 mg/ml. It is prepared by standard techniques of a pharmaceutical chemist. For example, lyophilized tPA can be reconstituted with an appropriate buffer, tPA in solution can be dialyzed against such appropriate buffer or the pH of a tPA solution can be adjusted with acid, salt or buffer. Thus, the parmaceutical dosage unit of the invention includes such dosage unit which has been lyophilized so that upon reconstitution with an aqueous solvent, a pharmaceutical dosage unit of the invention in solution form is achieved. Conveniently, the final steps of tPA purification can be carried out in a suitable, buffered aqueous solution at pH 3.5-5.5.

The pharmaceutical dosage unit of the invention is prepared so as to deliver to a patient, especially a human patient, in need of thrombolytic therapy an effective amount of tPA parenterally, especially intravenously, intracoronarily and perhaps, intraarterially. The precise concentration of tPA in the dosage unit as well as the precise volume of a given pharmaceutical dosage unit will depend upon the indication and the severity of the condition. Optimization of a given dosage unit can be carried out in accordance with standard pharmaceutical and medical practice. For acute myocardial infarction (acute MI), a patient will typically receive a total dosage of 25 to 125 mg, preferably about 70 to 90 mg, over a period of 10 to 180 minutes, preferably about 30 to 90 minutes. For example, Collen et al., Circulation, Volume 70, page 1012 - (1984), report treatment of patients suffering MI by intravenous infusion of 0.5 to 0.75 mg/kg, or (35-53 mg/70 kg person), over 30 to 120 min. The formulation reported by Collen et al. was contained in 10 ml vials, each containing 0.5 mg/ml of tPA. Sobel et al., Circulation, Volume 69, page 983 - (1984), report infusion of 200-400 x 10³ International Units (IU) of tPA to patients suffering MI. Van der Werf et al., N. Engl. J. Med. , Volume 310, page 609 (1984), report intravenous (i.v.) and intracoronary (i.c.) administration of doses of tPA ranging from about 3 x 10⁵ IU/15 min i.c. to about 1.4 x 10⁶ IU/35 min i.v. to MI patients. Robinson, WO84-01786, disclose treatment for a medium size thrombus by administering 0.10 to 1.0 mg/kg of a modified tPA lacking at least part of the carbohydrate portion of the native molecule by infusing up to 8 doses.

For patients suffering acute MI, the pharmaceutical dosage unit typically will be a bolus or injection form, i.e., a sachette, ampoule, vial or syringe containing up to 10 ml of tPA in a concentration of 1 to 15 mg/ml for ready administration. Such bolus-type injection can be followed with an intravenous infusion form containing 0.1 to 15 mg/ml of tPA. For other indications, for example, evolving MI, deep vein thrombosis (DVT) and pulmonary embolism (PE), the pharmaceutical dosage unit will typically be such intravenous infusion form in, for example, an i.v. bag or bottle for convenient connection to an intravenous cannula.

Thus, a preferred pharmaceutical dosage unit is a bolus form which can be stored in a vial, sachette, ampoule or syringe for ready usage in the event of emergency. Such dosage unit preferably contains about 1 to 10 ml, more preferably 2 to 6 ml, of 1 to 10 mg/ml, more preferably 2-6 mg/ml, of tPA. Such bolus form dosage unit is formulated to contain 2 to 50 mg, preferably 5-30 mg and more preferably 10-20 mg, of tPA for administration in a single injection. Another preferred pharmaceutical dosage unit is an intravenous infusion form which can be stored in a large (100 to 1000ml) container. Such unit preferably contains about 0.1 to 10 mg/ml, more preferably 0.5 to 5 mg/ml, of tPA, such that 10-1000 mg, preferably 50-500 mg, of tPA can be contained in a single infusion container. A third preferred embodiment of the invention combines both features, for example, a vial, syringe or other bolus-type container connected as a leader to an infusion form.

Lyophilized tPA can contain a pharmaceutically acceptable buffer in lyophilized form for eventual reconstitution in unbuffered solution, or, as when the lyophilization vehicle is ammonium acetate, the lyophilized product can be reconstituted in buffered solution. In any event, the lyophilized product can be stored in a sterile ampoule or other container for dispensation with a sterile container of a solution for reconstitution and eventual or immediate intravenous administration by injection or infusion. Thus, in a fourth preferred embodiment, the invention is a kit comprising one or more sterile containers of tPA in lyophilized form and one or more separate sterile containers of solution for reconstitution. The solution for reconstitution, the amount of tPA and the amount of solution for reconstitution in a single kit are selected so as to provide a final reconstituted product having 0.1 to 15 mg/ml at a pH selected in accordance with this invention. A preferred solution for reconstitution is a 5% solution of dextrose in water. The amounts of tPA and solution for reconstitution, preferably, are selected to provide a final reconstituted product, or dosage unit, which is the same as the preferred liquid injectable or infusable forms described above. Thus, a kit for preparing a preferred bolus form comprises, in one or more sterile containers, 2 to 50 mg, preferably 5 to 30 mg, of tPA and, in one or more second containers, 1 to 10 ml, preferably 2 to 6 ml, of the solution for reconstitution such that upon combining the contents of the two containers, the resulting product comprises tPA at a concentration of 1 to 10 mg/ml, preferably 2-6 mg/ml. A kit for preparing the preferred infusion form comprises, in one or more sterile containers, 10 to 1,000 mg, preferably 50 to 500 mg, of tPA and in one or more other sterile containers, 100 to 1000 ml of solution for reconstitution such that upon combining the contents of the containers, the resulting product comprises tPA at a concentration of 0.1-10 mg/ml, preferably 0.5-5 mg/ml. Preferably, all tPA for one dosage unit is contained in a single container and all solution for reconstitution for such dosage unit is contained in a second single container. A kit for preparing a pharmaceutical dosage unit of the invention can comprise a sterile multicompartment container, e.g., a two compartment container in which one compartment contains lyophilized tPA and a second compartment contains the solution for reconstitution, the two compartments being separated by a breakable barrier such that the contents of each can be aseptically mixed by breaking the barrier. Such kits are exemplified by the Univial® sterile two compartment vial (Abbot Laboratories, North Chicago, Illinois) and by the Hypak Liqui/Dry™ syringe (Becton Dickinson, Rutherford, New Jersey). Label or insert instructions indicating the quantities of tPA and solution for reconstitution and a protocol for administration can be included.

EXAMPLES

The following Examples 1-9 illustrate exemplary pharmaceutical dosage units of the invention.

EXAMPLE 1

A sterile syringe is filled with a sterile solution of

```
tPA                                        5 mg
100 mM acetic acid/ammonium acetate        5 ml
NaCl                                       0.5% (to adjust to
                                                isotonicity)
pH                                         4.0
```

EXAMPLE 2

A sterile syringe is filled with a sterile solution of

```
tPA                                        50 mg
60 mM acetic acid/sodium acetate            5 ml
dextrose                                    3% (to adjust to
                                                isotonicity)
chlorobutanol                              0.5 %
pH                                          3.5
```

EXAMPLE 3

A sterile syringe is filled with a sterile solution of

```
tPA                                        5 mg
100 mM citric acid/sodium citrate         10 ml
NaCl                                       0.13% (to adjust to
                                                 ·isotonicity)
methyl paraben                             0.1%
pH                                         5.5
```

EXAMPLE 4

A sterile syringe is filled with a sterile solution of

```
tPA                                        5 mg
100 mM citric acid/sodium citrate         10 ml
mannitol                                   0.8% (to adjust to
                                                isotonicity)
methyl paraben                             0.1%
pH                                         5.5
```

EXAMPLE 5

A sterile container useful for intravenous infusion is filled with a sterile solution of

| | |
|---|---|
| tPA | 100 mg |
| 50 mM glutamic acid/sodium glutamate | 1000 ml |
| NaCl | 0.7% (to adjust to isotonicity) |
| pH | 4.5 |

## EXAMPLE 6

A sterile container useful for intravenous infusion is filled with a sterile solution of

| | |
|---|---|
| tPA | 500 mg |
| 50 mM adipic acid/sodium adipate | 500 ml |
| thimerosal | 0.005% |
| NaCl | 0.7% (to adjust to isotonicity) |
| pH | 4.0 |

## EXAMPLE 7

A sterile container useful for intravenous infusion is filled with a sterile solution of

| | |
|---|---|
| tPA | 1000 mg |
| 100 mM acetic acid/ammonium acetate | 100 ml |
| dextrose | 3% (to adjust to isotonicity) |
| pH | 4.0 |

## EXAMPLE 8

A kit is packaged comprising (1) a sterile vial containing 10 mg tPA which has been lyophilized in a vehicle comprising tPA (10 mg/ml) in 100 mM ammonium acetate (pH 4) and 30% mannitol and - (2) a sterile vial containing a solution for reconstitution which is 100 mM ammonium acetate, 10 ml, pH 4.0.

## EXAMPLE 9

A kit is packaged comprising (1) a sterile vial containing 1000 mg tPA which has been lyophilized in a vehicle comprising tPA (15 mg/ml) in 100 mM ammonium acetate (pH 4) and 19% glycine and (2) a sterile bottle containing a solution for reconstitution which is 100 mM ammonium acetate, 1000 ml, pH 4.0.

## EXAMPLE 10

One ml of a solution containing 10 mg/ml of tPA in 0.1 M ammonium acetate was added in a 20 ml lyophilization vial to 4 ml of each of two excipient solutions to prepare Formulations A and B, as follows.

|  | A<br>(mg/vial) | B<br>(mg/vial) |
|---|---|---|
| PVP (Plasdone C-30) | 64 | 64 |
| Monosodium L-glutamate | 16 | - |
| Adipic acid | 6.4 | 6.4 |
| tPA | 10 | 10 |
| pH (Bulk solution) | 4.3 | 4.2* |

*pH was adjusted with Sodium Hydroxide

The formulations were lyophilized as follows. The vials were frozen overnight on a -50° shelf. The drying cycles were: (1) -25°C, 100 um Hg (13 MPa), 84 hr; (2) 0°C, 100 um Hg (13 MPa); 8 hr; and (3) 25°C, 10-30 um Hg (1.3 -3.9 MPa), 24 hr.

A control solution of tPA (1.0 mg/ml in 0.1 M ammonium acetate) was stored at 5°C. A first experimental sample (I) was stored in lyophilized form for 23 days at -15°C and then was reconstituted and assayed by S-2251 assay after 24 hours at 5°C, 25°C and 40°. Storage of the lyophilized plug at -15°C and of the reconstituted solution at 5°C simulates Day 0 conditions, i.e., reconstitution and assay immediately following lyophilization. A second experimental sample (II) was stored in lyophilized form for 23 days at 40°C and then was reconstituted and assayed by S-2251 assay after 24 hours at 4°C and 25°C. A third experimental sample (III) was stored in lyophilized form for 3 months at 40°C and then was reconstituted and assayed by S-2251 assay upon reconstitution and after 24 hours at 4°C and 25°C. All experimental samples were reconstituted to 1.0 mg/ml with 5% dextrose. The pH of the 23 day - (40°C) reconstituted formulations were A: ph 4.5 and B: pH 4.3. The osmolality of those formulations was A:298 mOsm and B:274 mOsm. Activity of the samples as a percentage of the control is reported in the following table.

|  | Sample I | | | Sample II | | Sample III | | |
|---|---|---|---|---|---|---|---|---|
|  | 24 hrs | | | 24 hrs | | 0 hrs | 24 hrs | |
|  | 5°C | 25°C | 40°C | 4°C | 25°C | - | 4°C | 25°C |
| A | 93 | 99 | 90 | 96 | 91 | 91 | 91 | 90 |
| B | 99 | 90 | 93 | 90 | 95 | 91 | 93 | 91 |
| Control | 100[1] | | | 100[1] | | 100[2] | 100[3] | |

[1] 6.87 x 10$^5$ IU/mg
[2] 6.08 x 10$^5$ IU/mg
[3] 5.67 x 10$^5$ IU/mg

EXAMPLE 11

Aliquots of two tPA formulations, A and B, prepared substantially as described above, were loaded into 5 ml vials such that each vial contained:

|  | A | B |
|---|---|---|
| PVP (Plasdone C-30) | 6.4 mg | 6.4 mg |
| L-aspartic acid monosodium | 1.5 mg | - |
| Adipic acid | 0.7 mg | 0.2 mg |
| tPA | 1.0 mg | 1.0 mg |
| pH (Bulk solution) | 4.4 | 4.1* |
| Total volume per vial | 0.5 ml | 0.5 ml |

*pH was adjusted with sodium hydroxide

The vials were lyophilized substantially as described above. One set was then stored at -15°C until 7 days after lyophilization to simulate Day 0 conditions, i.e., reconstitution and assay immediately following lyophilization, and assayed upon reconstitution and 24 hours after reconstitution at room temperature (20-25°C) (Day 0). Additional sets of vials were stored at 40°C and then were similarly reconstituted and assayed seven days after lyophilization (Day 7), three weeks after lyophilization (Week 3), and one month after lyophilization (Month 1). All lyophilized plugs were reconstituted with water to about pH 4.2 -4.6. Activity at each point was compared to that of a control formulation ·containing 1 mg/ml of tPA in 0.1 M ammonium acetate buffer, pH 4.0, stored at 5°C. Results are reported below as a percentage of the control.

|  | Day 0 | | Day 7 | | Week 3 | | Month 1 | |
|---|---|---|---|---|---|---|---|---|
|  | 0 hr | 24 hr | 0 hr | 24 hr | 0 hr | 24 hr | 0 hr | 24 hr |
| A | 107 | 93 | 100 | 97 | 98 | 97 | 91 | 97 |
| B | 104 | 97 | 107 | 92 | 103 | 100 | 92 | 94 |

EXAMPLE 12

Substantially as described in Examples 10 and 11, above, 10 ml vials were prepared containing:

| | |
|---|---|
| Gelatin | 5 mg |
| Monosodium L-glutamate | 5 mg |
| Mannitol | 15 mg |
| Sodium chloride | 1.7 mg |
| tPA | 0.1 mg |
| Total volume per vial | 1.2 ml |

The vials were lyophilized, and were reconstituted with 1.0 ml of 0.1 M ammonium acetate - (pH 4), and assayed at Day 0 (simulated). The vials stored at 40°C were assayed at Day 10, Day 16 and Month 1. Activity was compared to that of a control formulation (0.1 mg/ml of tPA in 0.1 M ammonium acetate buffer, pH 4, 5°C) and is reported below as a percentage of the activity of the control.

| Day 0 | | Day 10 | | Day 16 | | Month 1 | |
|---|---|---|---|---|---|---|---|
| 0 hr | 24 hr | 0 hr | 24 hr | 0 hr | 24 hr | 0 hr | 24 hr |
| 89 | 82 | 105 | 83 | 91 | 83 | 99 | 78 |

## EXAMPLE 13

Substantially as described in Examples 10 and 11, above, 5 ml vials were prepared containing:

| | |
|---|---|
| PVP (Plasdone C-30) | 6.4 mg |
| Monosodium L-glutamate | 1.6 mg |
| Adipic acid | 0.4 mg |
| tPA | 1.0 mg |
| pH (Bulk sodium) | 4.4 |
| Total volume per vial | 0.5 ml |

The vials were lyophilized and then were reconstituted with 1.0 ml of 0.1 M ammonium acetate (pH 4) and assayed on Day 0 (simulated). The vials stored at 40°C were assayed at Day 8 and Month 1. Activity was compared to that of a control formulation (1 mg/ml of tPA, in 0.1 M ammonium acetate buffer, pH 4.0, 5°C) and is reported below as a percentage of the activity of the control.

| Day 0 | | Day 8 | | Month 1 | |
|---|---|---|---|---|---|
| 0 hr | 24 hr | 0 hr | 24 hr | 0 hr | 24 hr |
| 106 | 112 | 101 | 109 | 100 | 100 |

Examples 10, 11, 12 and 13, above, demonstrate enhanced stability through lyophilization and upon reconstitution of a tPA formulation containing a polymeric cryoprotective agent, particularly, polyvinylpyrrolidone and gelatin.

The above disclosure and examples fully disclose the invention and preferred embodiments thereof. However, the invention is not limited to the particular constructions illustrated herein but rather encompasses all modifications coming within the scope of the following claims.

## INITIAL DEGRADATION RATES

ACETATE SOLUTIONS

UREA SOLUTIONS

pH 4.5
pH 5.0
pH 5.5
pH 4.0 9.8 mg/ml
pH 4.0 0.39 mg/ml
pH 3.5
pH 7.5, 0.39 mg/ml
pH 7.5, 1.3 mg/ml

% ACTIVITY LEFT

TIME(DAYS)

■ pH 5.0
□ pH 4.5
△ pH 5.5
▲ pH 4.0, 9.8 mg/ml

● pH 4.0, 0.39 mg/ml
○ pH 3.5
◇ pH 7.5, 0.39 mg/ml
◆ pH 7.5, 1.3 mg/ml

## Claims

1. A sterile, pharmaceutical dosage unit of tPA for parenteral administration which comprises 0.1 to 15 mg/ml of tPA in a pharmaceutically acceptable, buffered aqueous solution at pH 3.5-.5.5.

2. The pharmaceutical dosage unit of claim 1 which is a bolus form within a container having 1 to 10 ml of tPA at a concentration of 1 to 10 mg/ml and in an amount of 2 to 50 mg.

3. The pharmaceutical dosage unit of claim 1 which is a bolus form within a container having 2 to 6 ml of tPA at a concentration of 2 to 6 mg/ml and in an amount of 5 to 30 mg.

4. The pharmaceutical dosage unit of claim 1 which is an infusion form within a continer having 100 to 1,000 ml of tPA at a concentration of 0.1 to 10 mg/ml of tPA and in an amount of 10 to 1000 mg.

5. The pharmaceutical dosage unit of claim 1 which is an infusion form within a container having 100 to 1,000 ml of tPA at a concentration of 0.5 to 5 mg/ml and in an amount of 50 to 500 mg.

6. The pharmaceutical dosage unit of claim 1 which further comprises a cryoprotective agent in an amount which is safe and effective in stabilizing the tPA.

7. The pharmaceutical dosage unit of claim 6 in which the cryoprotective agent is gelatin or polyvinylpyrrolidone.

8. The pharmaceutical dosage unit of claim 7 in which the cryoprotective agent is polyvinylpyrrolidone, 40,000 molecular weight, in an amount of 1 to 15 mg per ml of tPA.

9. The pharmaceutical dosage unit of claim 1, 2, 4, 6, 7 or 8 which has a pH of 4.0-5.0

10. The pharmaceutical dosage unit of claim 1, 2, 4, 6, 7 or 8 which is buffered to pH 4.0-5.0 with a pharmaceutically acceptable salt of acetic acid, adipic acid, succinic acid, citric acid, lactic acid, tartaric acid, aspartic acid or glutamic acid.

11. The pharmaceutical dosage unit of claim 10 which is buffered with sodium acetate or ammonium acetate.

12. The pharmaceutical dosage unit of claim 1, 2 or 4 which comprises 4 to 10 mg of polyvinylpyrrolidone, 40,000 molecular weight, per mg of tPA and in which the solution is buffered to pH 4.4-5.0 with adipic acid.

13. A kit which comprises one or more sterile containers of tPA in lyophilized form and one or more other sterile containers of solution for reconstitution, wherein a buffer is provided in one or both of the containers and wherein the buffer, the solution for reconstitution and the amounts of the tPA and of the solution for reconstitution are selected so as to provide, upon combining the contents of the containers, a reconstituted product which is a pharmaceutical dosage unit for parenteral administration comprising 0.1 to 15 mg/ml of tPA in a buffered pharmaceutically acceptable solvent at pH 3.5-5.5.

14. The kit of claim 13 which comprises, in one or more sterile containers, 2 to 50 mg of tPA and, in one or more other sterile containers, 1 to 10 ml of solution for reconstitution.

15. The kit of claim 13 which comprises, in one or more sterile containers, 5 to 30 mg of tPA and, in one or more second sterile containers, 2 to 10 ml of solution for reconstitution.

16. The kit of claim 13 which comprises, in one or more sterile containers, 10 to 1,000 mg of tPA and, in one or more second sterile containers, 100 to 1000 ml of solution for reconstitution.

17. The kit of claim 13 which comprises, in one or more sterile containers, 50 to 500 mg of tPA and, in one or more second sterile containers, 100 to 1000 ml of solution for reconstitution.

18. The kit of claim 13 which comprises a cryoprotective agent in the one or more containers of tPA or in the one or more containers of solution for reconstitution or in both such that the reconstituted product contains an amount of the cryoprotective agent which is safe and effective in stabilizing the tPA.

19. The kit of claim 18 in which the cryoprotective agent is gelatin or polyvinylpyrrolidone.

20. The kit of claim 19 in which the cryoprotective agent is polyvinylpyrrolidone, 40,000 molecular weight, in an amount of 1 to 15 mg per mg of tPA.

21. The kit of claim 13, 14, 16, 18, 19 or 20 in which the reconstituted product has a pH of 4.0-5.0.

22. The kit of claim 13, 14, 16, 18, 19 or 20 in which the buffer is a pharmaceutically acceptable salt of acetic acid, adipic acid, succinic acid, citric acid, lactic acid, tartaric acid, aspartic acid or glutamic acid to buffer the reconstituted product to pH 4.0-5.0.

23. The kit of claim 22 in which the buffer is sodium acetate or ammonium acetate.

24. The kit of claim 13, 14 or 16 in which the one or more containers of tPA comprises 4 to 10 mg of polyvinylpyrrolidone, 40,000 molecular weight, per mg of tPA and adipic acid such that upon reconstitution the pH of the reconstituted product is 4.4-5.0

25. A lyophilisate comprising tPA and a pharmaceutically acceptable buffer in lyophilised form which upon reconstitution gives a pharmaceutically acceptable dosage unit according to any one of claims 1 to 12.

26. A lyophilisate according to claim 25 which upon reconstitution with water at neutral pH gives a pharmaceutically acceptable dosage unit according to any one of claims 1 to 12.

27. A lyophilisate according to claim 26 in which the buffer is adipic acid and a pharmaceutically acceptable salt thereof.

28. A process for preparing a sterile pharmaceutical dosage unit of tPA for parenteral administration which comprises dissolving tPA in a pharmaceutically acceptable buffered solvent at pH 3.5 to 5.5 to a concentration of 0.1 to 15 mg of tPA per ml of solution, optionally in the presence of a cryoprotective agent.

29. A process for the preparation of a sterile pharmaceutical dosage unit of tPA for parenteral administration which comprises lyophilising a bulk aqueous formulation containing 0.1 to 15 mg/ml tPA buffered to pH 3.5 to 5, optionally in the presence of a cryoprotective agent, and then reconstituting the lyophilised formulation with an aqueous solvent acceptable for parenteral administration whereby the pH of the formulation remains within the range of 3.5 to 5.

30. The process of claim 28 or 29 wherein the dosage unit is buffered with one or more of adipic acid, glutamic acid, citric acid, acetic acid, succinic acid, lactic acid, tartaric acid and aspartic acid and a salt thereof, and wherein the cryoprotective agent is polyvinylpyrrolidone or gelatin.

Claims for Contracting State : AT.

1. A process for preparing a sterile pharmaceutical dosage unit of tPA for parenteral administration which comprises dissolving tPA in a pharmaceutically acceptable buffered solvent at pH 3.5 to 5.5 to a concentration of 0.1 to 15 mg of tPA per ml of solution, optionally in the presence of a cryoprotective agent.

2. A process for the preparation of a sterile pharmaceutical dosage unit of tPA for parenteral administration which comprises lyophilising a bulk aqueous formulation containing 0.1 to 15 mg/ml tPA buffered to pH 3.5 to 5, optionally in the presence of a cryoprotective agent, and then reconstituting the lyophilised formulation with an aqueous solvent acceptable for parenteral administration whereby the pH of the formulation remains within the range of 3.5 to 5.

3. The process of claim 1 or 2 wherein the dosage unit is buffered with one or more of adipic acid, glutamic acid, citric acid, acetic acid, succinic acid, lactic acid, tartaric acid and aspartic acid and a salt thereof.

4. The process of any one of claims 1 to 3 when carried out in the presence of a cryoprotective agent.

5. The process of claim 4 wherein the cryoprotective agent is polyvinylpyrrolidone or gelatin.

6. The process of any one of claims 1 to 5 in which the dosage unit is buffered to pH 4.4 to 5.0